(19) **Europäisches Patentamt**

**European Patent Office**

**Office européen des brevets**

(11) **EP 0 956 872 B1**

(12) **EUROPÄISCHE PATENTSCHRIFT**

(45) Veröffentlichungstag und Bekanntmachung des Hinweises auf die Patenterteilung:
**06.04.2005 Patentblatt 2005/14**

(51) Int Cl.⁷: **A61M 1/16**

(21) Anmeldenummer: **99109004.4**

(22) Anmeldetag: **06.05.1999**

(54) **Blutreinigungsmaschine**

Blood purification apparatus

Appareil de purification du sang

(84) Benannte Vertragsstaaten:
**AT BE DE DK ES FR GB IT SE**

(30) Priorität: **14.05.1998 DE 19821534**

(43) Veröffentlichungstag der Anmeldung:
**17.11.1999 Patentblatt 1999/46**

(73) Patentinhaber: **B. Braun Medizintechnologie GmbH**
**34212 Melsungen (DE)**

(72) Erfinder:
• **Röher, Otfried, Prof. Dr.**
**01139 Dresden (DE)**
• **Korth, Steffen**
**99086 Erfurt (DE)**
• **Müller, Friedrich A.**
**35792 Loehnberg (DE)**
• **Rath, Dieter**
**34212 Melsungen (DE)**

(74) Vertreter: **Selting, Günther, Dipl.-Ing. et al**
**Patentanwälte**
**von Kreisler, Selting, Werner**
**Postfach 10 22 41**
**50462 Köln (DE)**

(56) Entgegenhaltungen:
**EP-A- 0 498 324          US-A- 4 718 891**

**Beschreibung**

Die Erfindung betrifft eine Blutreinigungsmaschine mit einer Ultrafiltrationsvorrichtung, bei der die Ultrafiltrationsrate geregelt wird.

Bei einer Blutreinigungsbehandlung mittels Hämodialyse, Hämodiafiltration oder Hämofiltration wird das Blut des Patienten in einem extrakorporalen Kreislauf durch eine Ultrafiltrationsvorrichtung geleitet und anschließend dem Patienten wieder zugeführt. In der Ultrafiltrationsvorrichtung werden dem Blut durch Konvektion und bei der Hämodialyse und Hämodiafiltration zusätzlich durch Diffusion Körperflüssigkeit und Schadstoffe entzogen, um die Funktion der Nieren zu ersetzen. Nachfolgend werden die genannten Verfahren der extrakorporalen Blutreinigung in Anlehnung an den medizinischen Sprachgebrauch unter der Bezeichnung "Hämodialyse" zusammengefaßt. Der Körper des Patienten enthält zu Beginn der Behandlung infolge der teilweise oder vollständig ausgefallenen Nierenfunktion einen Überschuß an Körperflüssigkeit und darin gelösten Schadstoffen, der ihm während der Hämodialysebehandlung über den Blutkreislauf entzogen wird. Während der mehrstündigen Behandlung besteht deshalb die Gefahr, daß infolge des intensiven Flüssigkeitsentzuges bedrohliche Blutdruckabfälle (symptomatische Hypotonien) ausgelöst werden. Derartige Komplikationen erfordern sofortige therapeutische Maßnahmen zur Stabilisierung des Blutdruckes. Es ist üblich, hierzu physiologische Kochsalzlösung (0.9-prozentige Natriumchlorid-Lösung) als Substituat in den Blutkreislauf zu infundieren. Dadurch wird das Blutvolumen und damit der Blutdruck erhöht, gleichzeitig aber dem notwendigen Flüssigkeitsentzug entgegengewirkt. Dies kann vermieden werden, indem statt größerer Volumina (z.B. 200-500 ml) physiologischer Kochsalzlösung entsprechend kleinere Volumina einer hochprozentigen (z.B. 20-prozentigen) Natriumchlorid-Lösung infundiert werden. Ihre gegenüber dem Körpergewebe wesentlich höhere Konzentration an Natrium-Ionen bewirkt, daß mittels Osmose zusätzliche Körperflüssigkeit aus dem Körpergewebe in die Blutgefäße einströmt. Die Infusion einer hochprozentigen Natriumchlorid-Lösung in den Körper erfordert jedoch einen zusätzlichen Aufwand an der Blutreinigungsmaschine durch Vorsehen einer Infusionspumpe, die eine komplexe Regelung benötigt.

EP 0 661 065 A2 beschreibt ein Infusionssystem mit Regeleinrichtung, bei dem die Regeleinrichtung unscharfes Wissen mittels linguistischer Variablen und Methoden der Fuzzy-Logik verarbeitet. Mehrere qualitativ unterschiedliche Einflußkomponenten werden als unscharfes Wissen erfaßt und ausgewertet sowie unter Berücksichtigung ihrer semantischen Bedeutung in die automatische Regelung der Infusionsvorrichtung einbezogen. Dadurch gelingt es, bei einer Dialysebehandlung die Infusion einer zusätzlichen Flüssigkeit in das Blutsystem so zu regeln, daß die qualitativ unterschiedlichen Einflußkomponenten sowie die im Laufe der Behandlung eintretenden Veränderungen in ihrem komplexen Zusammenwirken entsprechend ihrer semantischen Bedeutung erkannt und in die automatische Infusionsregelung einbezogen werden.

EP 0 652 780 B1 beschreibt eine Vorrichtung zum Vermeiden einer Hypotonie bei Dialysepatienten, mit der die Infusion einer hypertonen Natriumchlorid-Lösung durch eine entsprechende Erhöhung der Natrium-Konzentration in der Dialysierflüssigkeit (Dialysat) ersetzt werden kann. Hierzu wird dem Patienten über das Dialysat Natrium zugeführt, sobald der Patient oder das medizinische Personal ein entsprechendes Signal auslöst. Dies ist jedoch erst dann möglich, wenn ein Blutdruckabfall für den Patienten zu bewußt wahrnehmbaren Beschwerden führt. Die Natrium-Dosierung erfolgt nach fest vorgegebenen Werten mittels eines einfachen Steuerungssystems. Eine automatische Regelung der Natrium-Dosierung in Abhängigkeit vom aktuellen Blutdruck ist nicht möglich.

In US-A-4,718,891 wird berichtet, dass es im Stand der Technik üblich ist, den Blutdruck eines Dialysepatienten in periodischen Intervallen zu überwachen und durch das medizinische Personal therapeutisch einzugreifen, wenn eine hypotensive Episode auftritt. Die in diesem Fall einzuleitenden Maßnahmen zur Bewältigung und Korrektur einer hypotensiven Episode umfassen die Injektion einer Natriumlösung in den Blutstrom des Patienten und/oder die Reduzierung der Extraktionsrate der Flüssigkeit.

EP 0 498 324 Al beschreibt eine Blutreinigungsvorrichtung mit einem in den Blutkreislauf geschalteten Dialysator, dessen Dialysat-Zufuhr von einer Regeleinrichtung geregelt ist. Die Regelung erfolgt in Abhängigkeit von dem Blutdruck, der mit einer Punktionsnadel im Blutgefäß gemessen wird.

Bei Unterschreilen eines Grenzwertes für den Blutdruck wird eine vorbestimmte Menge zusätzlicher Flüssigkeit infunchiert sowie die Flüssigkeits extraktion auf O reduziert.

Bei der extrakorporalen Blutwäsche ist es ferner bekannt, die Ultrafiltrationsrate durch sogenannte Ultrafiltrationsprofile an den Zustand des Patienten anzupassen. Hierbei kann jedoch lediglich für verschiedene Zeitabschnitte der Blutreinigungs-Behandlung der zugehörige Wert der Ultrafiltrationsrate vor Behandlungsbeginn eingestellt werden. Bei Bedarf können die vorgewählten Ultrafiltrationsraten auch während der Behandlung vom medizinischen Personal manuell verändert werden. Bei diesen Geräten handelt es sich um einfache Steuersysteme mit willkürlicher Vorgabe der Ultrafiltrationsraten. Die Geräte verfügen über keine Sensoren zur Erfassung von Veränderungen des Patientenzustandes während der Behandlung.

Weiterhin sind Vorschläge bekannt, zur Vermeidung einer zu starken Blutvolumenabnahme (Hypovolämie) während der Hämodialysebehandlung Sensoren für die Messung der Blutvolumenabnahme bzw. des relativen Blutvolumens oder des Volumenanteils des Blutplasmas oder des Volumenanteils der zellulären Blutbestandteile (Hämatokrit) zu verwenden. Die Ultrafiltrationsrate wird hierbei in Abhängigkeit von den aktuellen Meßwerten so verändert, dass vorgewählte Soll- oder Grenzwerte dieser Größen eingehalten werden. Diese Konzepte sind für eine wirksame automatische Blutdruckstabilisierung nicht geeignet, da sie das Blutdruckverhalten nicht direkt erfassen und mit den genannten Volumenparametern nur einen Teil der multifaktoriellen Ursachen berücksichtigen, die den komplexen Wirkmechanismen der hämodialyseinduzierten Hypotonie zugrundeliegen.

Aufgabe der Erfindung ist deshalb die Schaffung einer Blutreinigungsmaschine, die den sich verändernden Blutdruck des Patienten durch Regelung der Ultrafiltrationsrate automatisch stabilisiert.

Die Lösung dieser Aufgabe erfolgt erfindungsgemäß mit den im Patentanspruch 1 angegebenen Merkmalen.

Nach der Erfindung wird bei einer Ultrafiltrationsvorrichtung die Ultrafiltrationsrate in Abhängigkeit von dem Blutdruck und ferner in Abhängigkeit von mindestens einem vom Blutdruck abgeleiteten Blutdruck-Trendwert mittels einer Fuzzy-Regelung geregelt. Die Ultrafiltrationsrate ist das Flüssigkeitsvolumen, welches über das Membransystem der Ultrafiltrationsvorrichtung pro Zeiteinheit dem Patienten entzogen wird. Die Ultrafiltrationsrate wird von der Regeleinrichtung nicht nur vorgegeben, sondern auch in Abhängigkeit von patientenbezogenen Werten automatisch verändert. Dabei hat sich ergeben, daß das Blutdruckverhalten eine sehr aussagefähige Größe darstellt. Während einer Dialysebehandlung kann infolge des Flüssigkeitsentzugs aus dem Blutsystem eine Blutdruckabsenkung (Hypotonie) eintreten. Das erfindungsgemäße Regelsystem kann solchen Blutdruckänderungen in geeigneter Weise begegnen, indem im Falle einer zu starken Blutdruckabsenkung die Ultrafiltrationsrate verringert wird. Zwar ist generell für die gesamte Behandlung ein durchschnittlicher Wert für die Ultrafiltrationsrate vorgegeben, jedoch wird dieser Wert durch die blutdruckabhängige Regelung verändert, wenn der Blutdruckverlauf eine Situation signalisiert, die einen Eingriff erfordert. Ein besonderer Vorteil besteht darin, daß der Eingriff aus einer Veränderung der Ultrafiltrationsrate besteht, so daß eine Infusion von Natriumchlorid-Lösung nicht erforderlich ist. Die hohen Kosten für eine Infusionspumpe können daher eingespart werden. Dies schließt allerdings nicht aus, daß im Falle eines extremen Behandlungsverlaufs Medikamente beispielsweise manuell injiziert werden. In bestimmten Behandlungsfällen kann es zweckmäßig sein, gemeinsam mit der Ultrafiltrationsrate auch eine Erhöhung der elektrischen Leitfähigkeit der Dialysierflüssigkeit oder eine Infusion einer hochprozentigen oder isotonen Infusionslösung mittels einer programmierbaren Infusionspumpe in die Regelung einzubeziehen.

Es liegt im Rahmen der Erfindung, für die Fuzzy-Regelung noch weitere physiologische Eingangsgrößen zu verwenden, z.B. die Änderung des Blutvolumens des Patienten seit Behandlungsbeginn (relatives Blutvolumen), die Änderung des Blutplasmavolumens (relatives Blutplasmavolumen) oder des Volumens der zellulären Blutbestandteile (Hämatokrit), die Änderung der Ionen-Konzentration oder der elektrischen Leitfähigkeit des Blutes, die Temperatur des Blutes oder der Dialysierflüssigkeit.

Das normale Ultrafiltrationsprofil, das entsteht, wenn die Eingangsgrößen des Fuzzy-Reglers keine abnormalen Werte annehmen, variiert gemäß einer vorteilhaften Weiterbildung der Erfindung in Abhängigkeit vom Ultrafiltratvolumen. Das Ultrafiltratvolumen ist das Integral über die bisherige Ultrafiltrationsrate und gibt das Flüssigkeitsvolumen an, das dem Körper bisher insgesamt entzogen wurde. Alternativ hiervon kann das Ultrafiltrationsprofil auch zeitabhängig oder von einer weiteren physiologischen Eingangsgröße abhängig sein. Hierfür kommen beispielsweise die Änderung der Ionen-Konzentration oder der elektrischen Leitfähigkeit des Blutes in Betracht. In jedem Fall ist das Ultrafiltrationsprofil aber kein vorgegebenes starres Profil, sondern es wird von dem Meßwert des Blutdrucks und weiteren Eingangsgrößen beeinflußt.

Gemäß einer bevorzugten Weiterbildung der Erfindung empfängt der Fuzzy-Regler als Eingangsgrößen den Blutdruck und einen Kurzzeit-Blutdruck-Trendwert und einen Langzeit-Blutdruck-Trendwert. Beide Trendwerte gehen in die semantische Domäne Blutdruck ein und ermöglichen eine Beurteilung des aktuellen Blutdruckwertes unter Einbeziehung vorausschauender Schlußfolgerungen.

Im folgenden wird unter Bezugnahme auf die Zeichnungen ein Ausführungsbeispiel der Erfindung näher erläutert.

Es zeigen:

Fig. 1        eine schematische Darstellung des Regelprinzips der Blutreinigungsmaschine,

Fig. 2        eine Kurve des Blutdruckverlaufs zur Erläuterung der Berechnung des Kurzzeit-Blutdruck-Trendwertes,

Fig. 3        eine Kurve des Blutdruckverlaufs zur Erläuterung der Ermittlung des Langzeit-Blutdruck-Trendwertes,

Fig. 4        ein Zeitdiagramm von Blutdruckverlauf und Ultrafiltrationsrate,

Fig. 5        ein Diagramm der Fuzzy-Regelung,

Fig. 5a       Schaubild der semantischen Domäne Hypotonie einschließlich ihrer Subdomänen,

Fig. 5b       Schaubild der semantischen Domäne Plasmavolumen einschließlich ihrer Subdomänen,

Fig. 5c    Schaubild der semantischen Domäne Ultrafiltration einschließlich ihrer Subdomänen,

Fig. 6    die Zugehörigkeitsfunktionen (Fuzzy-Sets) von drei Variablen, die als Entscheidungsmerkmale im ersten Fuzzy-Regler verwendet werden, und

Fig. 7    die Ermittlung des Schwerpunktes und Momentes der aktiven Zustände.

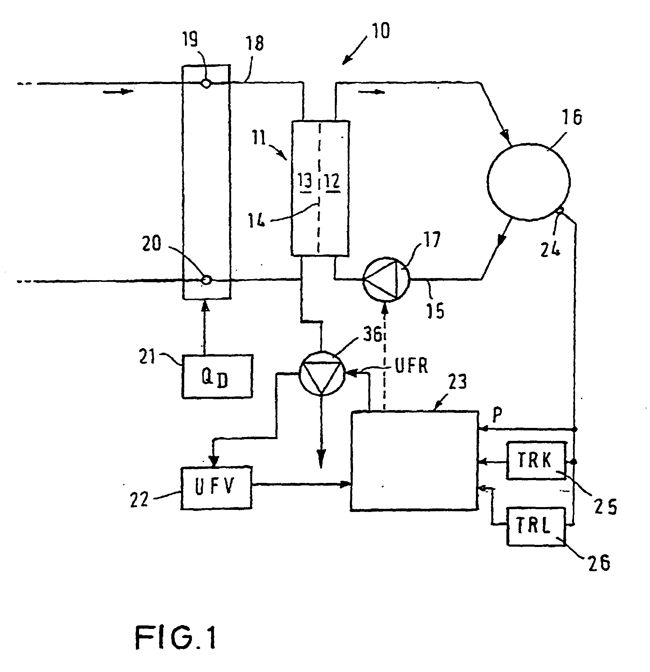

In Fig. 1 ist eine Blutreinigungsmaschine 10 dargestellt. Der Begriff Blutreinigungsmaschine soll umfassend verstanden werden, in dem Sinne, dass er sowohl eine Maschine für die Hämodialyse wie auch für die Hämofiltration bzw. für die Hämodiafiltration umfaßt.

Die Blutreinigungsmaschine 10 enthält eine Ultrafiltrationsvorrichtung 11 mit einer Primärkammer 12 und einer Sekundärkammer 13, die durch eine Membran 14 voneinander getrennt sind. Die Primärkammer 12 ist Bestandteil eines Blutkreislaufs 15, bei dem Blut, über das arterielle System des Patienten 16 entnommen wurde, in der Ultrafiltrationsvorrichtung 11 gereinigt und anschließend über das venöse System dem Patienten 16 zurückgeführt wird. In dem Blutkreislauf 15 befindet sich eine Pumpe 17. Diese ist als volumetrische Pumpe ausgebildet, d.h. die Fördermenge dieser Pumpe entspricht der Antriebsgeschwindigkeit und ist steuerbar.

Die Sekundärkammer 13 der Ultrafiltrationsvorrichtung 11 befindet sich in einem Dialysierflüssigkeitsweg 18, in dem Dialysierflüssigkeit gepumpt wird. Die Dialysierflüssigkeit kommt von einem (nicht dargestellten) Vorratsbehälter, nimmt in der Ultrafiltrationsvorrichtung 11 zusätzliche Substanzen aus dem Blut auf und wird dann zu einem (nicht dargestellten) Ablauf gepumpt. In dem Dialysierflüssigkeitsweg ist vor und hinter der Sekundärkammer 13 jeweils eine Durchflußkammer 19 bzw. 20 angeordnet, welche die Durchflußrate an der betreffenden Stelle regelt. Die Durchflußkammer 19 und die Durchflußkammer 20 haben die gleiche Förderrate. Über die volumengesteuerte Ultrafiltrationspumpe 36 wird die gewünschte Ultrafiltrationsmenge abgezogen. Der Dialysierflüssigkeitsfluß QD wird von einer Recheneinheit 21 konstantgehalten. Die Ultrafiltrationsrate UFR wird einer Recheneinheit 22 zugeführt, die das Zeitintegral über die Ultrafiltrationsrate bildet und so das Ultrafiltratvolumen UFV berechnet, also dasjenige Flüssigkeitsvolumen, das seit Beginn der Behandlung die Membran 14 passiert hat.

Die Regelung der Ultrafiltrationsrate erfolgt durch die Regeleinrichtung 23, die Steuersignale für die Förderrate der Pumpe 36 liefert. Die Förderrate der Pumpe wird in der Weise eingestellt, daß sich eine gewünschte Ultrafiltrationsrate ergibt.

Die Regeleinrichtung 23 empfängt ferner das Blutdrucksignal P eines Blutdruckmessers 24, der am Körper des Patienten 16 angebracht ist. Der Blutdruckmesser weist z.B. eine aufblasbare Manschette auf, die um den Oberarm des Patienten gelegt ist, und führt in vorgegebenen Intervallen von beispielsweise 5 min. nicht-invasive Blutdruckmessungen aus.

Der Blutdruckwert P wird ferner einer Recheneinheit 25 zugeführt, die einen Kurzzeit-Blutdruck-Trendwert TRK berechnet, und einer weiteren Recheneinheit 26, die einen Langzeit-Blutdruck-Trendwert TRL berechnet. Beide Trendwerte TRK und TRL werden in der Regeleinrichtung 23 verarbeitet.

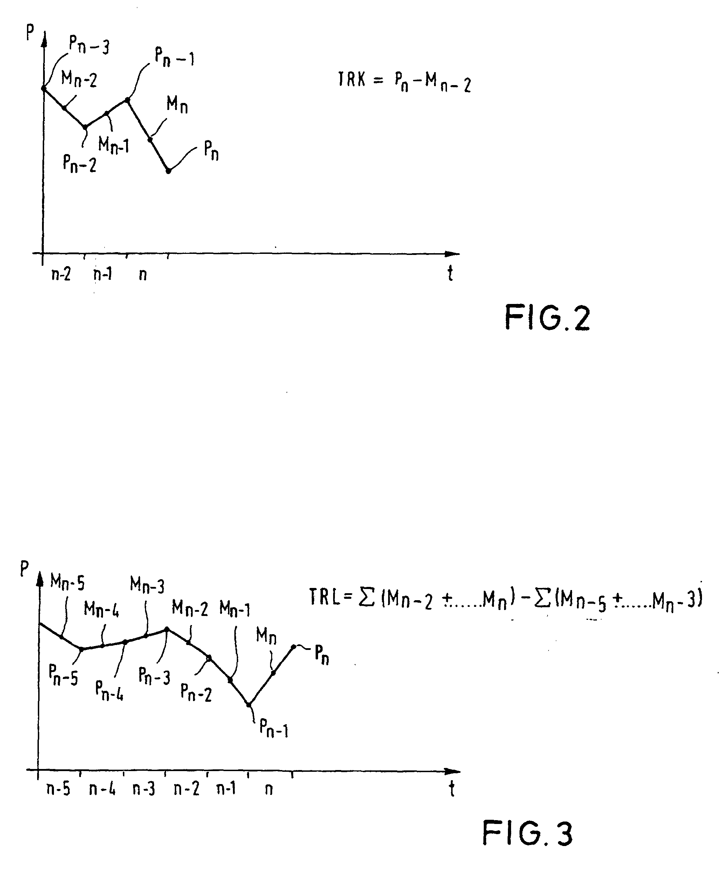

Fig. 2 zeigt die Berechnung des Kurzzeit-Blutdruck-Trendwerts TRK. Längs der Abszisse ist die Zeit t aufgetragen und längs der Ordinate der Blutdruck P. Die Zeit t ist in Intervalle n-2,n-1,n von 5 min. unterteilt. Am Ende eines jeden Intervalls erfolgt eine Blutdruckmessung, wobei der Blutdruckwert $P_{n-2}$, $P_{n-1}$ und $P_n$ festgestellt wird. Der arithmetische Mittelwert $M_{n-2}$ des Blutdrucks in dem Intervall n-2 ist der Mittelwert zwischen den Blutdruckwerten $P_{n-3}$ und $P_{n-2}$.

Der Kurzzeit-Trendwert am Ende des Intervalls n berechnet sich zu:

$$TRK = P_n - M_{n-2}.$$

Bei dem dargestellten Beispiel ist TRK negativ, d.h. der Kurzzeit-Trendwert signalisiert einen Blutdruckabfall. Ist dieser Blutdruckabfall größer als ein vorgegebener Grenzwert, erfolgt ein Regeleingriff.

Anhand von Fig. 3 wird die Berechnung des Langzeit-Trendwertes TRL verdeutlicht. Hier sind ebenfalls längs der Abszisse die Intervalle der Blutdruckmessung von n-5 bis n dargestellt und längs der Abszisse ist der Blutdruck P aufgetragen. Für den Langzeittrend werden die Mittelwerte $M_{n-5}$ ... $M_n$ ausgewertet. Diese Mittelwerte der einzelnen Intervalle werden aus den Blutdruckmeßwerten P, die dieses Intervall begrenzen, rechnerisch ermittelt.

Der Langzeittrend TRL des Blutdrucks berechnet sich zu:

$$TRL = \Sigma \, (M_{n-2} + ... M_n\,) - \Sigma \, (M_{n-5} + ... M_{n-3}).$$

Es wird also die Summe einer bestimmten Anzahl von Mittelwerten zusammenhängender jüngerer Intervalle gebildet und hiervon wird die Summe der Mittelwerte zusammenhängender älterer Intervalle subtrahiert. Ist der Wert TRL positiv, so entspricht dies einer tendenziellen Erhöhung des Blutdrucks; ist er negativ, entspricht dies einer abfallenden Tendenz.

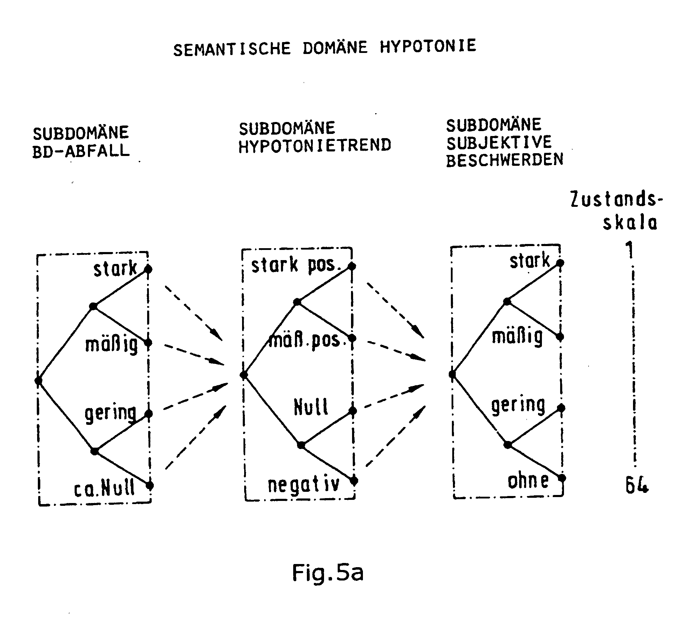

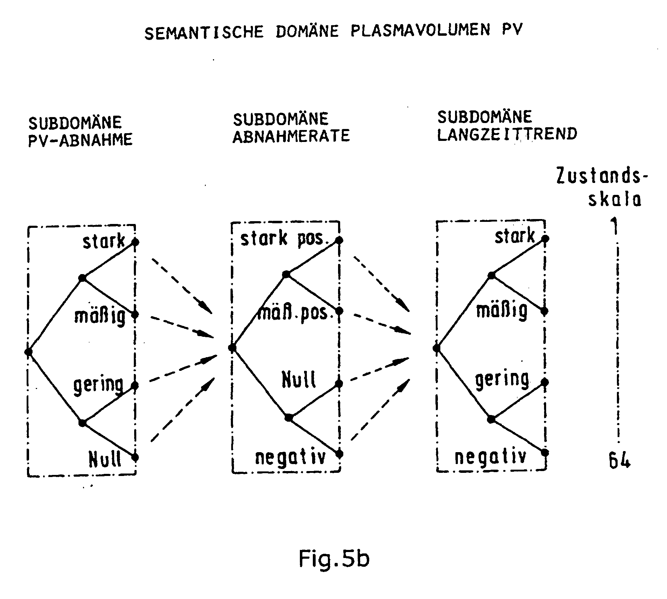

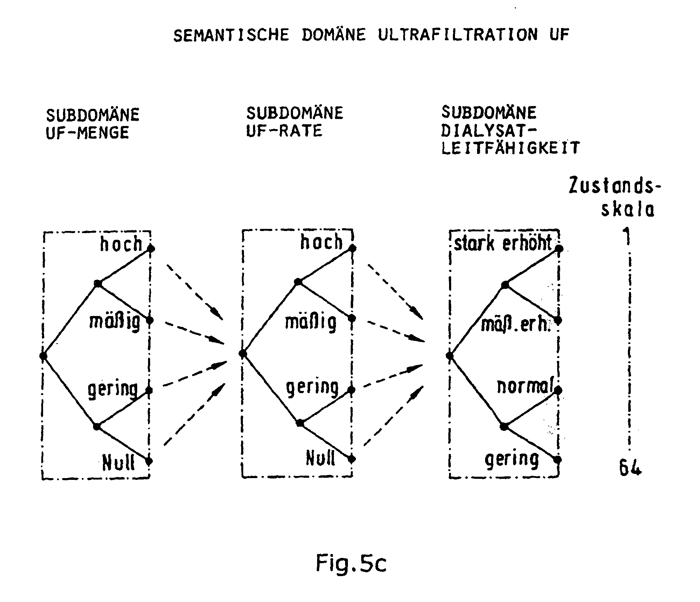

Der Aufbau der Regeleinrichtung 23 ist in Fig. 5 dargestellt. Diese Regeleinrichtung führt eine Fuzzy-Regelung durch. Die Einzelheiten einer solchen Fuzzy-Regelung sind in EP 0 661 065 A2 beschrieben.

Die folgenden wesentlichen Passagen aus EP 0 661 065 sind einschließlich der relevanten Zeichnungen in die vorliegende Beschreibung aufgenommen. In den eingefügten Passagen regelt der Steuergrössenakkumulator eine Infusionspumpe während in der Erfindung eine Ultrafiltrationseinrichtung geregelt wird. Entsprechende Passagen sind somit in Analogie zu interpretieren.

Sämtliche von der Messwertverarbeitungseinheit erfassten und abgeleiteten Größen werden dem Semantikanalysator 30 zugeführt, der ihre inhaltliche Bedeutung für die verschiedenen semantischen Domänen ermittelt und die erste Hierarchie-Ebene der Regeleinrichtung darstellt. Da für die semantischen Zusammenhänge zwischen den Eingangsgrößen und den semantischen Domänen kein scharfes (deterministisches) medizinisches Wissen existiert, wird für die Semantikanalyse ein erster Fuzzy-Regler 31 verwendet.

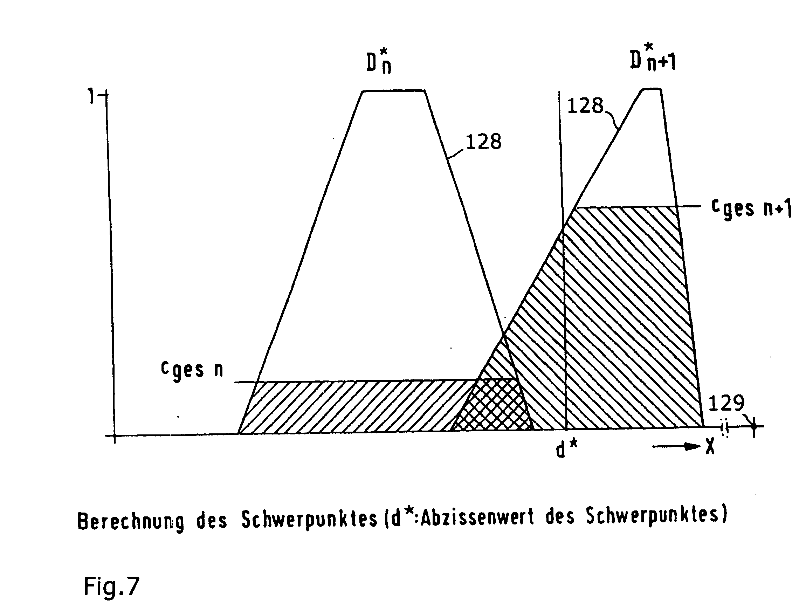

Fig. 5a zeigt eine schematische Darstellung der im ersten Fuzzy-Regler 31 verarbeiteten semantischen Domäne Hypotonie. Die Domäne ist in die Subdomänen Blutdruckabfall (BD-Abfall), Hypotonietrend und subjektive Beschwerden aufgeteilt und enthält somit 64 ($4^3$) verschiedene Zustände. Die auf dieser Grundlage entwickelte Regelbasis des Fuzzy-Reglers besteht dementsprechend aus einer Zustandsskala mit 64 Regeln. Jedem Zustand ist entsprechend seiner Bedeutung für den Blutdruckverlauf eine durch die zugehörige Regel festgelegt Medikamentendosis $D^*_n$ in Form eines Fuzzy-Sets zugeordnet, wie es Fig. 7 am Beispiel zweier Regeln $D^*_n$ und $D^*_{n+1}$ zeigt. Die x-Achse stellt den gesamten Dosisbereich dar, über den sich die 64 Regeln entsprechend ihrer Dosis verteilen.

Die Vielfalt der blutdruckrelevanten Einflusskomponenten veranschaulichen die semantischen Domänen Plasmavolumen in Fig. 5b und Ultrafiltration in Fig. 5c, wobei in beiden Domänen im Gegensatz zu Fig. 5a der Blutdruck selbst nicht als Entscheidungskriterium auftritt.

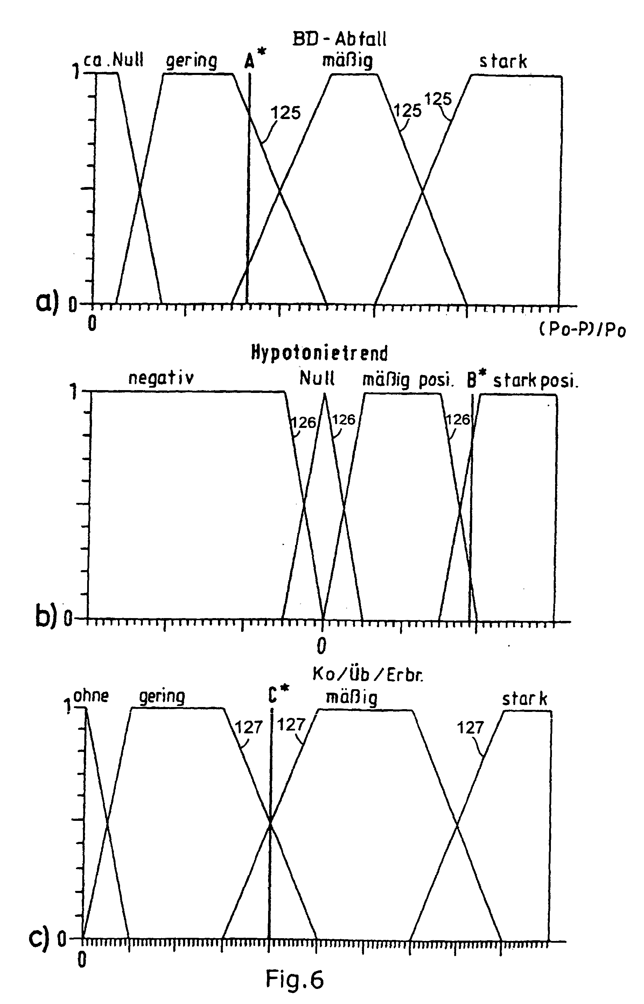

Fig. 6 zeigt am Beispiel der Zugehörigkeitsfunktionen von drei verschiedenen Entscheidungskriterien, wie die Fuzzy-Sets für unscharfes Wissen im Fuzzy-Regler 31 gespeichert sind. In Fig. 6a sind die Zugehörigkeitsfunktionen 125 für das Entscheidungskriterium Blutdruckabfall (BD-Abfall) dargestellt. Entlang der Abszisse ist der relative Blutdruckabfall $(P_o-P)/P_o$ aufgetragen, wobei bei $P_o$ der vorgewählte Soll-Blutdruck und P der gegenwärtig gemessene Blutdruck sind. Entlang der Ordinate sind die Zugehörigkeitswerte c in einer Skala von "0" bis "1" aufgetragen. Dem Entscheidungskriterium BD-Abfall sind vier linguistische Variablen als Merkmalsintervalle in Form von Fuzzy-Sets zugeordnet, nämlich die Intervalle "ca. Null", "gering", "mäßig" und "stark". Jedes dieser Merkmalsintervalle ist trapezförmig, wobei die Trapeze symmetrisch oder asymmetrisch sein können und sich gegenseitig überlappen.

Bei einem bestimmten aktuellen Blutdruckabfall, der in Fig. 6a als A* angegeben ist, ergeben sich aus den Schnittpunkten der Linie A* mit den Zugehörigkeitsfunktionen die Zugehörigkeitswerte für die jeweiligen linguistischen Variablen.

In Fig. 6b sind die Zugehörigkeitsfunktionen 126 für das Kriterium Hypotonietrend dargestellt. Die vier linguistischen Variablen sind "negativ", "Null", "mäßig positiv" und "stark positiv". Auch hier erfolgt die Fuzzyfizierung durch trapez- bzw. dreieckförmige Zugehörigkeitsfunktionen im Wertebereich 0...1. Der aktuelle Wert sei mit B* bezeichnet. Im dargestellten Beispiel ergeben sich aus den Schnittpunkten von B* Zugehörigkeitswerte >0 mit den Fuzzy-Sets "mäßig positiv" und "stark positiv".

Durch das dritte Entscheidungskriterium Subjektive Beschwerden in Fig. 6c werden Angaben über Kopfschmerz, Übelkeit und Erbrechen erfasst, die mit einer Bewertung ihres Stärkegrades in die Tastatur eingegeben werden. Der aktuelle Wert C* für dieses Entscheidungskriterium markiert im gezeigten Beispiel gleichgroße Zugehörigkeitswerte von 0,5 für die Merkmalsintervalle "gering" und "mäßig". Die Zugehörigkeitsfunktionen von Fig. 6c sind mit 127 bezeichnet.

Dem komplexen Zusammenwirken mehrerer Einflussgrößen aus unterschiedlichen semantischen Domänen wird im Semantikanalysator 30 durch einen mehrstufigen Inferenzprozess zur semantischen Bewertung der Domänen Rechnung getragen. Hierzu werden in der ersten Bewertungsstufe vom Fuzzy-Regler 31 die nach Fig. 6 ermittelten Zugehörigkeiten zu den einzelnen Merkmalsintervallen für die 64 möglichen Zustände mittels eines Mengenoperators, beispielsweise des Minimum-Operators, zu Gesamtzugehörigkeiten $c_{ges\,n}$ (n = 1 ...64) aggregiert. Alle Zustände, für die sich sowohl aus den aktuellen Fakten A* wie auch B* und C* Zugehörigkeitswerte >0 ergeben, werden dabei als aktivierte Zustände mit einer Gesamtzugehörigkeit >0 ausgewiesen.

Die erste semantische Bewertung der einzelnen Domänen wird hieraus durch Berechnung des Schwerpunktes und des Momentes der Gesamtzugehörigkeiten aller aktivierten Zustände der Domäne ermittelt. Das Moment einer semantischen Domäne ergibt sich aus der Lage des Schwerpunktes aller Gesamtzugehörigkeiten dieser Domäne.

Fig. 7 veranschaulicht die Berechnung des Schwerpunktes einer Domäne und seines Momentes am Beispiel von zwei aktivierten Zuständen $D^*_n$ und $D^*_{n+1}$, wobei n hier eine Zahl von 1 bis 64 auf der Zustandsskala ist und die Nummer des Zustandes in der betreffenden Domäne angibt. Jeder Zustand 128 ist durch ein trapezförmiges Fuzzy-Set definiert, dessen Lage auf der x-Achse seinen Dosisbereich markiert. Die beiden schraffierten Flächen entsprechen den Gesamtzugehörigkeiten $C_{ges\,n}$ und $C_{ges\,n+1}$, die aus den Schnittpunkten der aktuellen Fakten A*, B* und C* mit den Fuzzy-Sets der betreffenden Domäne ermittelt werden. Aus der Größe der schraffierten Flächen und ihrer Lage auf der x-Achse werden der Schwerpunkt und das Moment beider Zustände berechnet. d* ist der Abszissenwert des Schwerpunktes der beiden schraffierten Flächen, und das Moment ist das Produkt des Abstandes von d* vom Endwert 129 der Zustandsskala und dem Flächeninhalt der schraffierten Flächen.

Gemäß der vorliegenden Erfindung enthält die Regeleinrichtung 23 einen Semantik-Aralysator 30, dem die Eingangsgrößen P, TRK, TRL und UFV zugeführt werden. Der Semantik-Analysator enthält einen ersten Fuzzy-Regler 31, der die aus Zugehörigkeitsfunktionen ermittelten Zugehörigkeiten zu den einzelnen Merkmalsintervallen für insgesamt 64 mögliche Zustände mittels eines Mengenoperators zu Gesamtzugehörigkeiten $C_{ges\,n}$ (n = 1 ... 64) aggregiert. Eine erste semantische Bewertung der Domänen Hypotonie und Ultrafiltration wird durch Berechnung des Schwerpunktes und des Momentes der Gesamtzugehörigkeiten aller aktivierten Zustände der Domäne ermittelt.

Für die weiteren semantischen Bewertungsstufen verfügt der Semantik-Analysator 30 über eine Domänenbewertungs-Einheit 32, in der das ärztliche Wissen über den medizinischen Stellenwert der verschiedenen Zustände und die Schlussfolgerungen abgespeichert sind, die unter Berücksichtigung der ermittelten Momente der einzelnen Domänen zu ziehen sind. In der zweiten semantischen Bewertungsstufe erfolgt eine Gewichtung jeder einzelnen Domäne mit einem festgelegten Faktor. Im Ergebnis der zweiten semantischen Bewertungsstufe wird durch die Domänenbewertungs-Einheit 32 festgelegt, in welchem Umfang die Fuzzy-Regler der zweiten Hierarchie-Ebene an der Berechnung der von der Ultrafiltrationsvorrichtung 11 aufzubringenden Ultrafiltrationsraten beteiligt werden.

In der dritten semantischen Bewertungsstufe analysiert die Domänenbewertungs-Einheit 32, ob Gesamtzugehörigkeiten $C_{ges\,n}$ existieren, die außerhalb eines vorgegebenen Feldes liegen. Diese dritte semantische Bewertungsstufe läßt sowohl Wichtungsfaktoren analog zur zweiten Bewertungsstufe wie auch kontroverse Wichtungen zu, beispielsweise wenn zusätzlich zum Blutdruckverlauf weitere physiologische Messgrößen als Eingangsgrößen einbezogen werden und wenn ein stark aktivierter Zustand aus ärztlicher Sicht eine ausschließlich oder vorwiegend plasmavolumengesteuerte Ultrafiltration erfordert.

Gemäß Fig. 5 sind in der zweiten Hierarchie-Ebene die beiden Fuzzy-Regler 33,34 vorhanden, die ihre domänenspezifischen Inferenzergebnisse automatisch mit dem jeweiligen Wert für die semantische Gesamtbewertung der betreffenden Domäne multiplizieren. Der Fuzzy-Regler 33 ist der semantischen Domäne Hypotonie zugeordnet und der Fuzzy-Regler 34 der semantischen Domäne Ultrafiltration. Der Steuergrößenakkumulator 35 faßt die Ergebnisse der einzelnen Domänen zusammen und bildet daraus die Gesamt-Steuergröße für die Ultrafiltrationsvorrichtung 11. Hierzu wird die Summe aller semantischen Bewertungen gleich 100 gesetzt und die Inferenzergebnisse der einzelnen Fuzzy-Regler 33,34 werden mit dem entsprechenden Prozentanteil ihrer Domäne multipliziert. Die Summe der auf diese Weise normierten Prozentanteile aller Domänen gibt unmittelbar die von der Ultrafiltrationsvorrichtung aufzubringende bzw. durch entsprechende Einstellung der Pumpen 17,36 zu realisierende Ultrafiltrationsrate an.

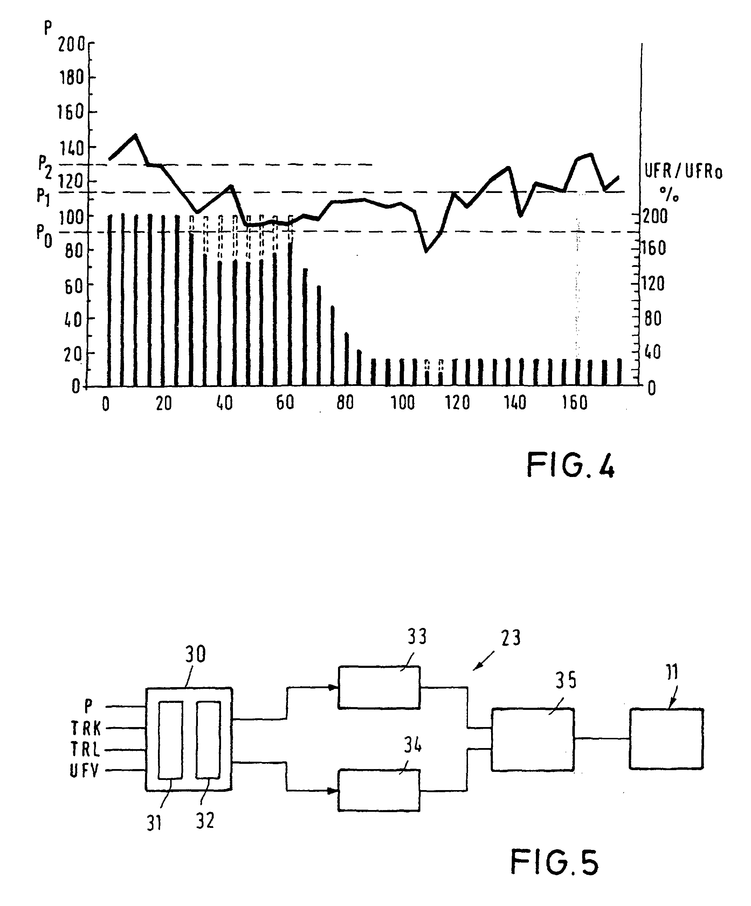

Fig. 4 zeigt die Auswirkung der beschriebenen Regelung auf die Ultrafiltrationsrate. Im unteren Teil des Diagramms ist die relative Ultrafiltrationsrate UFR/UFRo dargestellt, wobei UFRo die mittlere Ultrafiltrationsrate ist. Wenn beispielsweise über einen Zeitraum von 4 h insgesamt 4 l Flüssigkeit durch Ultrafiltration abgezogen werden sollen, beträgt die mittlere Ultrafiltrationsrate UFRo 1 l/h. In Abhängigkeit vom Blutdruckverlauf wird zu Beginn der Behandlung eine wesentlich höhere Ultrafiltrationsrate angewendet, die beispielsweise das Doppelte von UFRo beträgt und zur Erzielung einer Reserve an Ultrafiltratvolumen genutzt wird. Im Anschluß an die Anfangsphase wird die Ultrafiltrationsrate automatisch auf niedrigere Werte herabgesetzt, die den Blutkreislauf des Patienten weniger belasten. Der hohe Wert in der Anfangsphase ist dadurch gerechtfertigt, daß der Patientenkörper in dieser Phase noch sehr viel überschüssige Flüssigkeit enthält, so daß eine intensivere Ultrafiltration in der Regel gut toleriert wird. Mit fortschreitender Behandlungsdauer besteht jedoch die Gefahr, daß im Zusammenhang mit dem inzwischen entzogenen Ultrafiltratvolumen und mit der gegenüber UFRo erhöhten Ultrafiltrationsrate das Risiko eines Blutdruckabfalles zunehmen würde.

Im oberen Teil des Diagramms ist für eine Hämodialysebehandlung der Gesamtverlauf des Blutdrukkes P dargestellt, der automatisch in Intervallen von fünf Minuten wie üblich in mmHg gemessen wird. Der untere Grenzwert Po markiert den für jeden Patienten individuell vorzuwählenden Grenzblutdruck, bei dessen Unterschreiten die automatische Regelung der Ultrafiltrationsrate unbedingt aktiviert wird. Im Blutdruckbereich zwischen den Grenzwerten $P_0$ und $P_1$ wird die automatische Regelung nur dann aktiviert, wenn die Regeleinrichtung im Ergebnis einer semantischen Analyse des Blutdruckverlaufes Anzeichen eines sich anbahnenden Blutdruckabfalles erkennt.

Bei der dargestellten Kurve des Blutdruckverlaufs wird die Ultrafiltrationsrate in der ebenfalls angegebenen Weise verändert, und zwar sowohl in Abhängigkeit von dem Absolutwert des Blutdrucks P als auch in Abhängigkeit von den Trendwerten TRK und TRL. Das in Fig. 4 dargestellte und durch die gestrichelten Linien ergänzte Ultrafiltrationsprofil stellt das in der Regeleinrichtung 23 vorgespeicherte Profil dar. Die gestrichelten Linien, die die Werte der normierten Ultrafiltrationsrate nach oben ergänzen, geben die Ultrafiltrationsrate in dem Fall an, daß der Blutdruck hinreichend hoch wäre und nicht die Tendenz zu einem stärkeren Abfall zeigen würde, also den vorprogrammierten Verlauf der Ultrafiltrationsrate bei stabilem Blutdruckverlauf.

Dieses Profil wird durch die tatsächliche Entwicklung des Blutdrucks in der dargestellten Weise modifiziert. Bei der dargestellten Situation ist in mehreren Intervallen eine Reduzierung der Ultrafiltrationsrate erforderlich, so daß das Ultrafiltratvolumen zu einem bestimmten Zeitpunkt geringer ist als dasjenige Ultrafiltratvolumen, das nach dem gespeicherten Profil bei stabilem Blutdruckverlauf bereits entzogen sein könnte. Da das gespeicherte Ultrafiltrationsprofil in diesem Beispiel von dem Ultrafiltratvolumen abhängt, erfolgt der Übergang zu geringeren Ultrafiltrationsraten erst dann, wenn ein bestimmter Prozentsatz des insgesamt zu entfernenden Ultrafiltratvolumens erreicht ist, z.B. 70 %.

Eine andere Möglichkeit besteht darin, das in der Regeleinrichtung 23 vorgespeicherte Ultrafiltrationsprofil für stabilen Blutdruckverlauf nicht in Abhängigkeit vom Ultrafiltratvolumen zu programmieren, sondern in Abhängigkeit von der Behandlungszeit. Weitere Alternativen sehen vor, daß das Ultrafiltrationsprofil für stabilen Blutdruckverlauf in Abhängigkeit von anderen Eingangsgrößen vorgegeben wird. Solche Eingangsgrößen sind insbesondere das relative Blutvolumen, das relative Blutplasmavolumen und das Volumen der zellulären Blutbestandteile (Hämatokrit), die Ionen-Konzentration und die elektrische Leitfähigkeit des Blutes oder die Temperatur des Blutes oder der Dialysierflüssigkeit. In allen Alternativen werden die vorgespeicherten Ultrafiltrationsprofile für stabilen Blutdruckverlauf durch die tatsächliche Entwicklung des Blutdruckverlaufes in der beschriebenen Weise modifiziert.

**Patentansprüche**

1. Blutreinigungsmaschine mit einer Ultrafiltrationsvorrichtung (11) und einer Regeleinrichtung (23), die einen Fuzzy-Regler (31) enthält, der als Eingangsgrößen den Messwert eines Blutdruckmessers (24) und mindestens einen Blutdruck-Trendwert empfängt, **dadurch gekennzeichnet, dass** die Regeleinrichtung (23) die Ultrafiltrationsrate regelt und ein Ultrafiltrationsprofil enthält, das von einer physiologischen Eingangsgröße oder dem Ultrafiltratvolumen oder der Zeit abhängig ist und in Abhängigkeit vom Blutdruck-Meßwert und in Abhängigkeit von mindestens einem Blutdruck-Trendwert variiert wird.

2. Blutreinigungsmaschine nach Anspruch 1 **dadurch gekennzeichnet, dass** die Regeleinrichtung (23) einen Semantik-Analysator (30) aufweist, der einen ersten Fuzzy-Regler (31) enthält, welcher die Eingangsgrößen zu semantischen Domänen zusammenfaßt und in jeweils einer Zustandsskala anordnet, wobei der erste Fuzzy-Regler (31) jede Domäne anhand gespeicherter Fuzzy-Sets und der Zustandsskala analysiert, indem er für jeden einzelnen Zustand der Zustandsskala den Zugehörigkeitswert ($C_{ges}$) ermittelt, dass eine Bewertungseinheit (32) die Zugehörigkeitswerte domänenspezifisch wichtet, wobei die Bewertungseinheit für jede Domäne eine semantische Bewertung erzeugt, dass für jede Domäne ein weiterer Fuzzy-Regler (33,34) vorgesehen ist, der aus den Zugehörigkeitswerten der Domäne und aus der semantischen Bewertung eine domänenspezifische Steuergröße erzeugt, und dass ein Steuergrößenakkumulator (35) die domänenspezifischen Steuergrößen mehrerer Domänen zu einer Gesamt-Steuergröße für die Ultrafiltrationsvorrichtung (11) zusammenfaßt.

3. Blutreinigungsmaschine nach Anspruch 1 oder 2, wobei die Regeleinrichtung (23) zur Regelung der Ultrafiltrationsrate zusätzlich Mittel zur Regelung der Erhöhung der elektrischen Leitfähigkeit der Dialysierflüssigkeit enthält.

4. Blutreinigungsmaschine nach einem der Ansprüche 1-3, wobei die Regeleinrichtung (23) zur Regelung der Ultrafiltrationsrate zusätzlich Mittel zur Regelung der Zudosierung einer hochprozentigen oder isotonen Infusionslösung durch eine Infusionspumpe enthält.

5. Blutreinigungsmaschine nach Anspruch 3 oder 4, wobei die Regeleinrichtung (23) das Ultrafiltrationsprofil in Abhängigkeit von der Erhöhung der elektrischen Leitfähigkeit der Dialysierflüssigkeit oder der Zudosierung einer hochprozentigen oder isotonen Infusionslösung verändert.

**Claims**

1. Blood purification apparatus comprising an ultrafiltration device (11) and a control means (23) including a fuzzy controller (31) which receives as input variables the measured value from a blood pressure meter (24) and at least one blood pressure trend value, **characterized in that** the control means (23) controls the ultrafiltration rate and includes an ultrafiltration profile which depends on a physiological input variable or the ultrafiltration volume or the time and is varied in dependence on the blood pressure measured value and in dependence on at least one blood pressure trend value.

2. Blood purification apparatus according to claim 1, **characterized in that** the control means (23) comprises a semantic analyzer (30) including a first fuzzy controller (31) which combines the input variables to form semantic domains and arranges them in one condition scale for each domain, wherein the first fuzzy controller (31) analyzes each domain on the basis of stored fuzzy sets and the condition scale by determining for each condition of the condition scale the membership value ($C_{ges}$),

an evaluation unit (32) carries out a domain-specific weighting of the membership values, wherein the evaluation unit performs a semantic evaluation for each domain,

for each domain a further fuzzy controller (33,34) is provided which forms a domain-specific control variable from the membership values of the domain and the semantic evaluation, and

a control variable accumulator (35) combines the domain-specific control variables of a plurality of domains into a overall control variable for the ultrafiltration device (11).

3. Blood purification apparatus according to claim 1 or 2, wherein the control means (23) for controlling the ultrafiltration rate comprises additional means for controlling the increase in the electrical conductivity of the dialysis fluid.

4. Blood purification apparatus according to one of claims 1-3, wherein the control means (23) for controlling the ultrafiltration rate comprises additional means for controlling the dosing of a highly concentrated or isotonic infusion solution by an infusion pump.

5. Blood purification apparatus according to claim 3 or 4, wherein the control means (23) varies the ultrafiltration profile in dependence on the increase in the electrical conductivity of the dialysis fluid or the dosing of a highly concentrated or isotonic infusion solution.

**Revendications**

1. Appareil de purification du sang doté d'un dispositif d'ultrafiltration (11) et d'un dispositif de régulation (23), qui contient un régulateur à logique floue (31), qui reçoit la valeur de la mesure de la tension artérielle (24) et au moins une valeur tendancielle de la tension artérielle, **caractérisé en ce que** le dispositif de régulation (23) régule la vitesse d'ultrafiltration et contient un profil d'ultrafiltration qui est dépendant d'une valeur d'entrée physiologique ou du volume d'ultrafiltration ou du temps, et qui varie en fonction de la valeur mesurée de la tension artérielle et en fonction au moins d'une valeur tendancielle de la tension artérielle.

2. Appareil de purification du sang selon la revendication 1, **caractérisé en ce que** le dispositif de régulation (23) présente un analyseur sémantique (30), qui contient un premier régulateur à logique floue (31), qui regroupe les valeurs d'entrée en domaines sémantiques et les agence à chaque fois en une échelle d'état, le premier régulateur à logique floue (31) analysant chacun des domaines au moyen de l'ensemble flou enregistré et de l'échelle d'état en déterminant pour chaque état différent de l'échelle la valeur d'appartenance ($C_{ges}$),

**en ce qu'**une unité d'estimation (32) mesure les valeurs d'appartenance spécifiques aux domaines, l'unité d'estimation pour chaque domaine produisant une estimation sémantique,

**en ce que** pour chaque domaine, est prévu un autre régulateur à logique floue (33,34) qui produit à partir des valeurs d'appartenance du domaine et à partir de l'estimation sémantique une grandeur de contrôle spécifique au domaine, et

**en ce qu'**un accumulateur des grandeurs de contrôle (35) regroupe les grandeurs de contrôle spécifiques aux domaines de plusieurs domaines en une grandeur de contrôle globale pour le dispositif d'ultrafiltration (11).

3. Appareil de purification du sang selon la revendication 1 ou 2, dans lequel le dispositif de régulation (23) pour la régulation de la vitesse d'ultrafiltration comprend en plus des moyens permettant de réguler l'augmentation de la conductance électrique du liquide de dialyse.

4. Appareil de purification du sang selon l'une quelconque des revendications 1 à 3, dans lequel le dispositif de régulation (23) pour la régulation de la vitesse d'ultrafiltration comprend en plus des moyens permettant de réguler l'ajout dosé par une pompe à perfusion d'une solution de perfusion à pourcentage élevé ou isotonique.

5. Appareil de purification du sang selon la revendication 3 ou 4, dans lequel le dispositif de régulation (23) modifie le profil d'ultrafiltration en fonction de l'augmentation de la conductance électrique du liquide de dialyse ou de l'ajout dosé d'une solution de perfusion à pourcentage élevé ou isotonique.

FIG.1

$$TRK = P_n - M_{n-2}$$

FIG.2

$$TRL = \sum (M_{n-2} + \ldots M_n) - \sum (M_{n-5} + \ldots M_n - 3)$$

FIG.3

FIG. 4

FIG.5

SEMANTISCHE DOMÄNE HYPOTONIE

Fig.5a

SEMANTISCHE DOMÄNE PLASMAVOLUMEN PV

Fig.5b

SEMANTISCHE DOMÄNE ULTRAFILTRATION UF

SUBDOMÄNE
UF-MENGE

SUBDOMÄNE
UF-RATE

SUBDOMÄNE
DIALYSAT-
LEITFÄHIGKEIT

Zustandsskala

1

hoch

mäßig

gering

Null

hoch

mäßig

gering

Null

stark erhöht

mäß.erh.

normal

gering

64

Fig.5c

14

Fig.6

Berechnung des Schwerpunktes (d*:Abzissenwert des Schwerpunktes)

Fig.7

EP 0 956 872 B1